(19) **Europäisches Patentamt**

**European Patent Office**

**Office européen des brevets**

(11) Veröffentlichungsnummer: **0 362 476**
**A2**

(12) **EUROPÄISCHE PATENTANMELDUNG**

(21) Anmeldenummer: **89110949.8**

(22) Anmeldetag: **16.06.89**

(51) Int. Cl.⁵: **C07C 51/487 , C07C 57/30 , C07B 57/00**

(30) Priorität: **19.07.88 DE 3824353**

(43) Veröffentlichungstag der Anmeldung:
**11.04.90 Patentblatt 90/15**

(84) Benannte Vertragsstaaten:
**AT BE CH DE ES FR GB IT LI LU NL SE**

(71) Anmelder: **PAZ ARZNEIMITTEL-ENTWICKLUNGSGESELLSCHAFT MBH**
**In der Schildwacht 13**
**D-6230 Frankfurt/Main 80(DE)**

(72) Erfinder: **Lukas, Helmut, Dr.**
**Taunusstrasse 30**
**D-6078 Neu-Isenburg(DE)**
Erfinder: **Schuster, Otto, Dr.**
**Kelkheimerstrasse 69**
**D-6230 Bad Soden(DE)**
Erfinder: **Rau, Günther, Dr.**
**Richard-Wagner-Strasse 48A**
**D-6239 Kriftel(DE)**

(74) Vertreter: **Schulze, Ilse, Dipl.-Chem.**
**Gaisbergstrasse 3**
**D-6900 Heidelberg(DE)**

(54) **Verfahren zur Trennung von Gemischen enantiomerer Arylpropionsäuren.**

(57) Es wird ein Verfahren beschrieben, bei dem Gemische enantiomerer Arylpropionsäuren getrennt und eine der enantiomeren Formen der Säuren gewonnen wird. Das Gemisch wird mit einer chiralen Base und einem inerten Lösungsmittel zu einem diastereomeren Salz umgesetzt und daraus das gewünschte Säure-Enantiomer abgetrennt. Die Umsetzung zum diastereomeren Salz erfolgt in einem polaren Lösungsmittel, das erhaltene Salz wird mehrfach umkristallisiert und das gereinigte Salz in verdünnter Mineralsäure gespalten.

EP 0 362 476 A2

EP 0 362 476 A2

## Verfahren zur Trennung von Gemischen enantiomerer Arylpropionsäuren

Die Erfindung betrifft ein Verfahren zur Trennung von Gemischen enantiomerer Arylpropionsäuren und zur Gewinnung einer der enantiomeren Formen der Säuren, bei dem das Gemisch mit einer chiralen Base in einem inerten Lösungsmittel zu einem diastereomeren Salz umgesetzt und daraus das gewünschte Säure-Enantiomer abgetrennt wird.

Es ist bekannt, daß die analgetische und antirheumatische Wirkung der Enantiomere höher ist als die der racemischen 2-Arylpropionsäuren. Dabei weist die eine enantiomere Form eine größere therapeutische Aktivität auf als die andere.

In der US-PS 4 209 638 ist ein Verfahren zur Erhöhung des Anteils eines gewünschten Enantiomers einer 2-Arylpropionsäure beschrieben. Dabei wird ein Gemisch aus einem Salz der 2-Arylpropionsäure und einem inerten flüssigen organischen Verdünnungsmittel auf mindestens 80°C erhitzt. Es wird so viel Salz verwendet, daß noch ein Teil im Verdünnungsmittel ungelöst bleibt. Es wird so lange erwärmt, bis ein Teil eines der optischen Isomeren der Säurekomponente durch Salzbildung in sein Enantiomer gespalten ist. Die Säurekomponente wird dann abgetrennt.

Dieses Verfahren erfordert neben einer erheblichen Menge an Lösungsmittel verhältnismäßig hohe Temperaturen, wobei ferner teilweise unter Anwendung von Druck gearbeitet werden muß. Trotzdem läßt die Reinheit des so gewonnenen Produktes zu wünschen übrig. Das Verfahren ist sowohl raum- als auch zeitaufwendig und stößt daher im großtechnischen Rahmen auf Schwierigkeiten.

Aufgabe der Erfindung war es, ein Verfahren der eingangs genannten Art zu schaffen, bei dem wesentlich günstigere Raum-Zeit-Faktoren als bei bekannten Verfahren erreicht werden, dabei aber gleichzeitig eine gute Ausbeute an optisch sehr reinem Produkt erhalten wird.

Diese Aufgabe wird durch ein Verfahren der eingangs genannten Art dadurch gelöst, daß man die Umsetzung zum diastereomeren Salz in einem polaren Lösungsmittel vornimmt, das erhaltene Salz durch mehrfache Kristallisation reinigt, das gereinigte Salz in verdünnter Mineralsäure spaltet und dabei die gewünschte Enantiomerform gewinnt.

Zweckmäßige Ausführungsformen des Verfahrens sind in den Unteransprüchen gekennzeichnet.

Es wurde überraschenderweise - und entgegen der allgemeinen Meinung - gefunden, daß die Verwendung von polaren Lösungsmitteln weitaus günstiger war als diejenige apolarer Lösungsmittel. Es wurde keineswegs eine wesentliche Menge Lösungsmittel benötigt, wie von der Fachwelt angenommen und es werden bei kleinstem Volumen und kürzester Reaktionszeit hoher Ausbeuten und ein sehr reines optisches Produkt erhalten.

Im wesentlichen wird wie folgt gearbeitet.

X Mol 2-Arylpropionsäure werden mit $\frac{X}{2}$ Mol der chiralen Base im entsprechenden Anteil Lösungsmittel versetzt und noch ca. 30 Min. nach der beendeten Zugabe der Base nachgerührt. Nach dem evtl. erforderlichen Abkühlen wird das diastereomere Salz vom Lösungsmittel abgetrennt und mehrfach, vorzugsweise 1 bis 4 mal, umkristallisiert. Das so gereinigte Diastereomer wird in Wasser suspendiert, mit Salzsäure gespalten und von der wässrigen Lösung abgetrennt.

Das Verhältnis zwischen diastereomerem Salz und Lösungsmittel liegt im Bereich von 11 : 0,2 bis 1 : 100, vorzugsweise bei 1 : 0,6 bis 1:15 (G/V).

Bevorzugt wird das Verfahren unter Rückfluß durchgeführt. bei empfindlichen Basen kann unter Schutzgas, beispielsweise Stickstoff gearbeitet werden.

Die Arbeitstemperaturen liegen zwischen Raumtemperatur und der jeweiligen Siedetemperatur der verwendeten Lösungen.

Das erfindungsgemäße Verfahren wird anhand zweier Beispiele näher erläutert.

Beispiel 1

115 kg Ibuprofen (537,4 Mol) wurden im Rührkessel in 300 l 2-Propanol unter Erwärmen gelöst. Zu der etwa 60°C heißen Lösung wurden 33,8 kg Phenylethylamin in 87 l 2-Propanol zugegeben. Nach beendeter Zugabe wurde die Lösung etwa 30 Minuten bei Siedehitze gerührt und unter Rühren auf Raumtemperatur abgekühlt. Das aus gefallene Diastereomere wurde abgepreßt und mit ca. 20 l eisgekühlten 2-Propanol gewaschen.
Ausbeute: 64,8 kg Diastereomer (71% d.Th.) (enantiomere Reinheit: 89%).

Dieses Diastereomer wurde aus 343 l Ethanol heiß umkristallisiert.
Ausbeute: 50,2 kg Diastereomer (55% d.Th.) (enantiomere Reinheit: 95%).

2

Dieses Diastereomer wurde aus 281 l Ethanol heiß umkristallisiert.
Ausbeute: 43,7 kg Diastereomer (47,9% d.Th.) (enantiomere Reinheit: 98%).
Dieses Diastereomer wurde aus 276 l Ethanol heiß umkristallisiert.
Ausbeute: 38,2 kg Diastereomer (42% d.Th.) (enantiomere Reinheit: >99,5%).

Das so gereinigte Diastereomer wurde unter Rühren in 300 l Wasser (dem.) suspendiert, mit 13 l Salzsäure (32%) versetzt, nach 2 Stunden abgepreßt, mit Wasser (dem.) neutralgewaschen und getrocknet. Endausbeute: 24,1 kg (42% d.Th.) (S)-(+)-Ibuprofen (enantiomere Reinheit: >99,5%).

Detaillierte Angaben sind in der folgenden Tabelle aufgeführt.

Dabei sind hier noch Ergebnisse mit Ketoprofen und Fluorbiprofen sowie Chinin und Chinchonidin angegeben.

| Beispiel | Säure | Base | Lösungsmittel | Volumenverhältnis [g Salz/ml Lösung] | Temperatur | Ausbeute | $[\alpha]_D$ der Säure [1] |
|---|---|---|---|---|---|---|---|
| 3 | I | 1 | Methylalkohol | 1: 1,4 – 1: 4,7 | RT–Siedetem. | ca. 30 % | + 60.4 |
| 4 | I | 1 | Ethylalkohol | 1: 1,6 – 1: 6,3 | RT–Siedetem. | ca. 42 % | + 60.2 |
| 5 | I | 1 | 2-Propanol | 1: 2,7 – 1:12 | RT–Siedetem. | ca. 60 % | + 60.1 |
| 6 | I | 1 | Ethylacetat | 1:16,2 – 1:40 | RT–Siedetem. | ca. 43 % | + 60.3 |
| 7 | I | 1 | Ethylacetat-Ethylalkohol (5+1) | 1: 5,5 – 1: 9 | RT–Siedetem. | ca. 53 % | + 60.4 |
| 8 | I | 1 | Dimethylformamid | 1: 3,1 – 1:11 | RT–Siedetem. | ca. 55 % | + 60.3 |
| 9 | I | 1 | Aceton | 1: 2,8 – 1:10 | RT–Siedetem. | ca. 48 % | + 60.3 |
| 10 | I | 1 | Dimethylsulfoxid | 1: 3,1 – 1:11 | RT–Siedetem. | ca. 41 % | + 60.4 |
| 11 | I | 1 | Monoglyme | 1: 3,1 – 1:11 | RT–Siedetem. | ca. 44 % | + 60.3 |
| 12 | I | 2 | Methylalkohol | 1: 0,7 – 1: 3 | RT–Siedetem. | ca. 20 % | + 60.4 |
| 13 | I | 2 | Isopropanol | 1: 1,0 – 1: 1,5 | RT–Siedetem. | ca. 32 % | + 60.2 |
| 14 | I | 2 | Ethylalkohol | 1: 0,63– 1: 1 | RT–Siedetem. | ca. 41 % | + 60,2 |
| 15 | I | 3 | Dimethylformamid | 1: 10,0 | RT–Siedetem. | ca. 48 % | + 60,3 |
| 16 | K | 1 | 2-Propanol | 1: 1,0 – 1: 1,5 | RT–Siedetem. | ca. 40 % | + 49,8 |
| 17 | F | 1 | 2-Propanol | 1: 1,0 – 1: 3,0 | RT–Siedetem. | ca. 42 % | + 44,7 |

[1]

       C = 10% in Methanol

Säure:  I = Ibuprofen,
          K = Ketoprofen,
          F = Fluorbiprofen

Basen:  1 = Phenylethylamin,
          2 = Chinin,
          3 = Chinchonidin

Beispiel 2

50 kg Ibuprofen (242.4 Mol) wurden im Rührkessel unter Erwärmen in 80 Liter 2-Propanol gelöst. Zu dieser Lösung wurden 29,4 kg (242.4 Mol) Phenylehtylamin in 30 Liter 2-Propanol gegeben. Nach beendeter Zugabe wurde die Lösung etwa 30 Minuten bei Siedehitze gerührt und anschließend auf ca. 2 bis 5° C abgekühlt. Das ausgefallene Diastereomere wurde abgepreßt und 2-mal mit je 20 Liter eisgekühltem 2-Propanol gewaschen.
Ausbeute: 39,7 kg Diastereomer (100% d. Ausbeute) enantiomere Reinheit: 80 %
Durch dreimaliges Umkristallisieren aus Ethanol und Spaltung mit Salzsäure gemäß Beispiel 1 wurde folgende Endausbeute erhalten:
12,5 kg (50% d. Ausbeute) enantiomere Reinheit: 98 %

## Ansprüche

1. Verfahren zur Trennung von Gemischen enantiomerer Arylpropionsäuren und zur Gewinnung einer der enantiomeren Formen der Säuren, bei dem das Gemisch mit einer chiralen Base in einem inerten Lösungsmittel zu einem diastereomeren Salz umgesetzt und daraus das gewünschte Säure-Enantiomer abgetrennt wird,
**dadurch gekennzeichnet,** daß man die Umsetzung zum diastereomeren Salz in einem polaren Lösungsmittel vornimmt,
das erhaltene Salz durch mehrfache Kristallisation reinigt,
das gereinigte Salz in verdünnter Mineralsäure spaltet und dabei die gewünschte Enantiomerform gewinnt.

2. Das Verfahren nach Anspruch 1, dadurch gekennzeichnet, daß man das racemische Gemisch der Arylpropionsäure oder ein Gemisch der beiden Enantiomere behandelt.

3. Verfahren nach Anspruch 1, dadurch gekennzeichnet, daß man ein Enantiomer als diastereomeres Salz abgetrennt und den verbleibenden Rückstand direkt aufgearbeitet oder wiederholt mit der chiralen Base behandelt um den enantiomeren Anteil zu erhöhen.

4. Verfahren nach Anspruch 1, dadurch gekennzeichnet, daß der Anteil der chiralen Base X/2 bis X Mol, bezogen auf X Mol Arylpropionsäure, beträgt.

5. Verfahren nach Anspruch 1, dadurch gekennzeichnet, daß die Umsetzung zum diastereomeren Salz bei Temperaturen zwischen Raumtemperatur und der jeweiligen Siedetemperatur des polaren Lösungsmittels durchgeführt wird.

6. Verfahren nach einem oder mehreren der Ansprüche 1 bis 5, dadurch gekennzeichnet, daß als polares Lösungsmittel niedere Alkohole, Ether oder Ester allein oder deren Gemische verwendet werden.

7. Verfahren nach Anspruch 1, dadurch gekennzeichnet, daß als chirale Base Phenylethylamin, Chinolinalkaloide oder Ephedrinbasen verwendet werden.

8. Verfahren nach Anspruch 1, dadurch gekennzeichnet, daß das Verhältnis zwischen diastereomeren Salz und Lösungsmittel im Bereich von 1 : 0,2 bis 1 : 100 liegt.

9. Verfahren nach Anspruch 1, dadurch gekennzeichnet, daß man unter Rückfluß arbeitet.

10. Verfahren nach Anspruch 1, dadurch gekennzeichnet, daß man unter Schutzgas arbeitet.

11. Verfahren nach Anspruch 1, dadurch gekennzeichnet, daß nach der ersten Fällung das Ibuprofen aus dem diastereomeren Salz freigesetzt wird und die weitere Anreicherung durch einfache Umkristallisation erzielt wird, indem die erste Kristallfraktion aus einem optisch aktiven Arylpropionsäuregemisch besteht und die Mutterlauge angereichert den entsprechenden optischen Antipoden enthält.